# FASCICULE DE BREVET EUROPEEN

(11) **EP 4 041 606 B1**
(45) Date de publication et mention de la délivrance du brevet: **19.07.2023**
(21) Numéro de dépôt: 20775665.1
(22) Date de dépôt: 25.09.2020
(51) Int. Cl.: B60W 40/08, B60W 50/14, B60W 30/14, A61B 5/16, G06V 10/82, G06N 3/08, G02B 27/00

(54) **DÉTERMINATION D'UN ÉTAT D'UTILISATEUR CONDUISANT ACTIVEMENT OU NON UN VÉHICULE AUTOMOBILE**
BESTIMMUNG EINES ZUSTANDES EINES BENUTZERS, DER AKTIV EIN MOTORFAHRZEUG FÄHRT ODER NICHT
DETERMINATION OF A STATE OF A USER ACTIVELY DRIVING A MOTOR VEHICLE OR NOT

(30) Priorité: 03.10.2019 FR 1910976
(43) Date de publication de la demande: 17.08.2022
(73) Titulaire: Valeo Vision, 93012 Bobigny Cedex (FR)
(72) Inventeur: GOSSELE, Frederic, 93012 Bobigny Cedex (FR); LAFAYSSE, Pierre Cesar, 93012 Bobigny Cedex (FR); THOUVENOT, Pierre Louis, 93012 Bobigny Cedex (FR)
(74) Mandataire: Valeo Visibility
(86) Numéro de dépôt international: PCT/EP2020/077010
(87) Numéro de publication internationale: WO 2021/063846

(56) Documents cités:
- EP-A2- 2 952 403
- WO-A1-2017/192260
- WO-A2-2017/195200
- DE-A1-102015 011 708
- KR-A- 20180 072 523

## Description

### Domaine technique

La présente invention concerne la détermination d'au moins un état d'un utilisateur dans un habitacle de véhicule automobile, en tant que personne conduisant activement ou non le véhicule.

On entend par « personne ne conduisant pas activement le véhicule » aussi bien :
- un passager du véhicule (qui n'est pas le conducteur),
- qu'un conducteur de véhicule autonome, ce véhicule étant autonome au-delà d'un seuil (par exemple au-delà de 80%).

### Technique antérieure

Une cause substantielle des accidents de la route est liée à l'utilisation de dispositifs de télécommunication (type smartphone ou autre). En particulier, lors de la réception de notifications, l'utilisateur est tenté de concentrer son attention sur l'écran de son smartphone. Une solution pour remédier à ce problème est de détecter que l'utilisateur est dans un véhicule en mouvement (par exemple à l'aide d'un module de géolocalisation dans le dispositif de télécommunication) et de filtrer toutes les communications entrantes à destination de son dispositif. Il s'agit toutefois d'une solution extrême car un utilisateur peut être simple passager dans le véhicule. En outre, avec la prolifération des véhicules autonomes, les conducteurs sont de plus en plus disponibles pour recevoir des notifications sur leur smartphone s'ils ne conduisent pas activement leur véhicule.

De manière plus générale, la présente divulgation vise alors à détecter si un utilisateur est dans un état courant où il conduit activement, ou non, un véhicule. Par exemple, il peut être possible alors de lui transmettre des notifications s'il est détecté comme ne conduisant pas activement le véhicule. A cet effet, la présente divulgation propose de suivre les mouvements de l'oeil de l'utilisateur (par une caméra typiquement mettant en oeuvre un suivi oculaire ou « eye tracking » en anglais). Il a été observé en effet qu'un utilisateur passager ou simplement ne conduisant pas activement le véhicule a tendance à balayer son regard vers le paysage qu'il regarde défiler. Au contraire, un conducteur actif du véhicule focalise son attention sur la route et son regard est davantage fixe. Ainsi la fréquence de balayage du regard peut être un critère pour déterminer, si elle est au-delà d'un seuil typiquement, qu'un utilisateur n'est pas conducteur actif.

Néanmoins, il a été observé que la fréquence de balayage du regard pouvait être propre à un utilisateur, et qu'elle pouvait varier d'un utilisateur à l'autre.

WO 2017/192260 A1 divulgue un système qui détecte l'état du conducteur et d'un passager et transfère le contrôle à la personne la plus concentrée.

KR 2018 0072523 A, EP 2 952 403 A2, DE 10 2015 011708 A1 et WO 2017/195200 A2 décrivent des systèmes qui détectent l'état du conducteur ou d'un occupant du véhicule basé entre autres sur la fréquence de mouvements oculaires.

### Résumé

La présente divulgation vient améliorer la situation.

Elle propose à cet effet un procédé de détermination d'au moins un état d'un utilisateur dans un habitacle de véhicule automobile, en tant que personne conduisant activement ou non le véhicule, dans lequel on prévoit un dispositif capteur de mouvements oculaires de l'utilisateur. En particulier, le procédé comporte :
- alimenter une intelligence artificielle avec des données issues du dispositif capteur pour reconnaitre au moins une fréquence de mouvements oculaires de l'utilisateur, une fréquence de mouvements oculaires au-delà d'un premier seuil caractérisant une visualisation par l'utilisateur d'un paysage défilant, et se distinguant d'une concentration de regard d'un conducteur de véhicule,
- déterminer une fréquence courante de mouvements oculaires de l'utilisateur et comparer la fréquence courante au premier seuil, et
- si la fréquence courante est supérieure au premier seuil, déclencher un signal de notification à destination de l'utilisateur.

Ainsi, l'usage d'une intelligence artificielle permet de déterminer par apprentissage et avec certitude pour un utilisateur donné, si ce dernier est effectivement en état de conduite active ou non par mesure de sa fréquence moyenne ou habituelle de mouvements oculaires en étant hors d'un état de conduite active. Plus précisément, cette fréquence des mouvements oculaires peut varier avec la vitesse du véhicule et être étalonnée en fonction de la vitesse pour un utilisateur donné, spécifique, sur lequel une étape préalable d'apprentissage a été mise en oeuvre pour alimenter l'intelligence artificielle précitée.

Une telle réalisation permet ainsi de déterminer de façon précise et certaine si l'utilisateur est en conduite active ou non, ce qui apporte de nombreux avantages :
- d'une part, on peut éviter l'affichage de notifications sur un dispositif communiquant de l'utilisateur lorsque ce dernier est détecté comme conduisant activement le véhicule, et ce même si le véhicule n'est pas en mouvement à grande vitesse,
- d'autre part, on peut laisser l'affichage des notifications reçues sur le dispositif communiquant de l'utilisateur lorsque ce dernier est détecté comme ne conduisant pas activement le véhicule.

Par « concentration de regard du conducteur de véhicule », on entend la concentration de regard, typiquement sur la route, qu'applique habituellement un conducteur de véhicule.

Par ailleurs et comme indiqué précédemment, on entend par « utilisateur ne conduisant pas activement le véhicule » aussi bien un passager du véhicule qui n'est pas le conducteur, qu'un conducteur de véhicule autonome au-delà d'un seuil (par exemple en situation d'autonomie au-delà de 80%).

Ainsi, dans une réalisation, si la fréquence courante précitée est supérieure au premier seuil, l'utilisateur est déterminé comme simple passager ou conducteur du véhicule sous un mode de conduite autonome à un niveau supérieur ou égal à 80%.

Comme indiqué précédemment à titre d'exemple, le signal de notification, précité, peut être transmis à un appareil communiquant de l'utilisateur. Cet appareil communiquant peut être de type smartphone, tablette, PC ou autre, connecté typiquement à un réseau cellulaire. Le signal de notification peut être reçu par ce réseau, respectivement par SMS, messagerie instantanée ou courriel.

Dans cette forme de réalisation, l'appareil communiquant peut être configuré pour :
- déterminer si l'utilisateur est dans un véhicule en mouvement, et
- dans ce cas, filtrer des notifications entrantes sur l'appareil communiquant,
   le procédé comportant en outre :
- déterminer si l'utilisateur n'est pas en situation de conduite active, et dans ce cas désactiver le filtrage des notifications entrantes sur l'appareil communiquant.

On entend indistinctement ici par « filtrer les notifications entrantes » aussi bien la possibilité de couper l'appareil communiquant du réseau cellulaire pour empêcher la réception de toute notification (par exemple en mode « avion »), que la possibilité de recevoir les notifications mais sans qu'elles s'affichent sur l'écran de l'appareil.

Par exemple, l'appareil peut comporter un module détecteur de mouvement de l'appareil (par exemple par géolocalisation ou autre) et déterminer ainsi, au-delà d'une vitesse seuil, que l'utilisateur est dans un véhicule en mouvement. Dans ce cas, ce module détecteur peut filtrer toutes les notifications entrantes sur l'appareil, mais aussi peut communiquer avec un circuit de traitement à bord du véhicule et connecté au capteur de mouvements oculaires, pour laisser arriver les notifications sur l'appareil si le circuit de traitement détermine que l'utilisateur n'est pas en situation de conduite active.

En complément ou en variante, le procédé peut comporter en outre :
- déterminer une position courante du véhicule, et
- déclencher des signaux de notifications pertinentes en fonction de la position courante du véhicule, à destination de l'appareil de l'utilisateur.

Par exemple, le véhicule peut comporter un module de détermination de position courante (de type GPS pour « Global Positioning System »), et un circuit de traitement connecté :
- au capteur de mouvements oculaires d'une part, pour mettre en oeuvre le procédé ci-avant, et
- au module précité de détermination de position courante d'autre part, pour déterminer une position courante du véhicule et déclencher des signaux de notifications pertinentes en fonction de la position courante du véhicule, à destination de l'appareil de l'utilisateur.

Il est possible ainsi de proposer une « visite guidée » du paysage à l'utilisateur pendant son trajet dans le véhicule.

Dans une réalisation, le dispositif capteur peut mesurer en outre une amplitude de mouvements oculaires de l'utilisateur, et le procédé comporte alors en outre :
- comparer une amplitude courante à une amplitude seuil et
- si l'amplitude courante est inférieure à l'amplitude seuil, déterminer une situation de mal des transports que subit l'utilisateur, et déclencher une action dans le véhicule pour soulager l'utilisateur.

Il a été observé en effet qu'une amplitude moindre des mouvements de l'utilisateur était corrélée à un mal des transports ressenti par l'utilisateur.

Là encore, le procédé peut mettre en oeuvre une intelligence artificielle pour détecter spécifiquement pour cet utilisateur un mal des transports ou un risque de mal des transports, le procédé comportant alors :
- alimenter une intelligence artificielle avec des données issues du dispositif capteur pour reconnaitre au moins une amplitude de mouvements oculaires de l'utilisateur, l'utilisateur étant en situation de mal des transports lorsque l'amplitude de mouvements oculaires mesurée est inférieure à ladite amplitude seuil.

L'action qui peut être déclenchée dans le véhicule peut comporter au moins l'un des éléments parmi :
- une réduction de vitesse du véhicule au moins dans des virages que prend le véhicule,
- une aspersion de senteurs dans l'habitacle du véhicule,
- une modification des conditions thermiques dans l'habitacle,
- une projection d'un scénario lumineux dans l'habitacle.

Dans une réalisation, le procédé peut comporter en outre :
- obtenir des coordonnées de géolocalisation courantes du véhicule pour identifier un parcours en cours du véhicule comme étant facteur de risque de déclenchement de mal des transports, et
- transmettre les coordonnées de géolocalisation courantes à un serveur de base de données avec un identifiant de mal des transports.

Dans cette réalisation, le serveur distant est préférentiellement capable de :
- recevoir des coordonnées de géolocalisations depuis une flotte de véhicules, avec un identifiant de mal des transports,
- estimer pour une pluralité de parcours possibles d'un point à un autre, des scores respectifs de risque de déclenchement de mal des transports, et
- transmettre à un ou plusieurs véhicules des données pour déterminer un parcours alternatif à un parcours à score élevé de risque de déclenchement de mal des transports.

La communication avec le serveur peut être assurée par l'appareil communiquant de l'utilisateur (smartphone ou autre) ou par module de communication cellulaire implanté dans le véhicule.

On peut prévoir en outre un accéléromètre (dans le véhicule, par exemple sur le smartphone de l'utilisateur) pour estimer une fréquence courante de cahots du véhicule, un parcours étant confirmé comme à risque de déclenchement de mal des transports si la fréquence courante de cahots dépasse une fréquence seuil.

Dans une réalisation, on peut prévoir en outre un capteur de rayonnement UV (pour « ultraviolet) et, en cas de détection d'une signature de rayonnement UV supérieure à un rayonnement seuil, ladite notification à destination de l'utilisateur comporte un message de sensibilisation au rayonnement UV.

Outre cette notification, il est possible de déclencher une action dans le véhicule en cas de détection de rayonnement UV parmi au moins : l'action d'un brumisateur, une ventilation, une climatisation, une opacification aux rayons UV des vitres du véhicule.

Par ailleurs, le capteur de rayonnement UV peut être un capteur d'intensité de lumière dans les longueurs d'onde UV ou simplement un capteur de température différentielle par rayonnement solaire dans l'habitacle.

La présente invention vise aussi un dispositif comportant un circuit de traitement et au moins un capteur de mouvements oculaires du type précité pour la mise en oeuvre du procédé ci-avant.

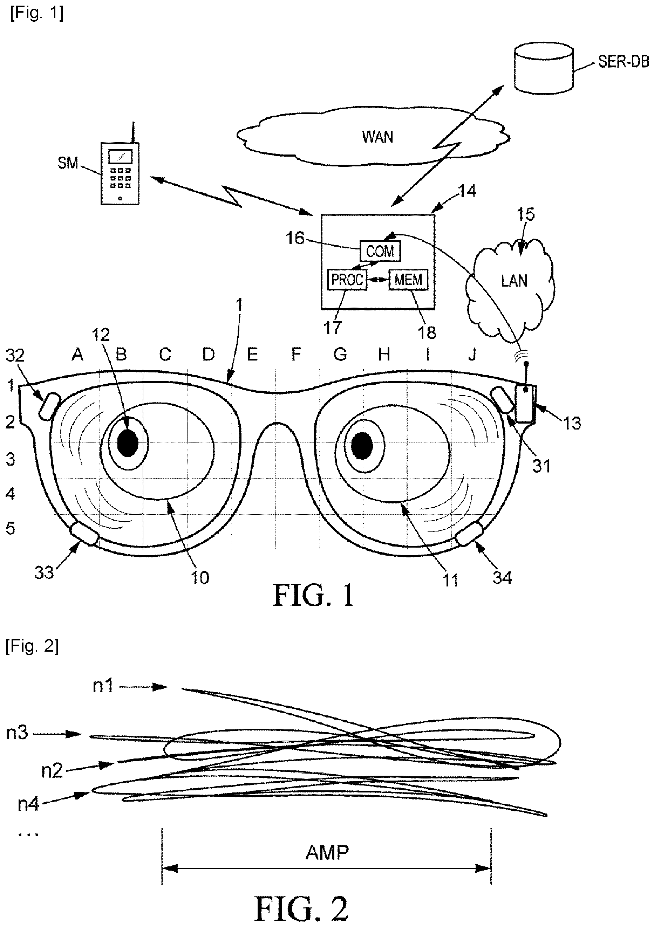

Dans un tel dispositif, le dispositif capteur de mouvements oculaires de l'utilisateur peut être monté sur une paire de lunettes connectée que porte l'utilisateur (comme illustré sur la figure 1 commentée plus loin). Cette paire de lunettes peut porter aussi l'accéléromètre précité, le détecteur de rayonnement UV, et/ou autres, voire aussi l'appareil communiquant pour des lunettes à verres informatifs.

En variante, le dispositif capteur de mouvements oculaires de l'utilisateur peut être monté sur une caméra aménagée dans l'habitacle du véhicule (face au conducteur par exemple), la caméra pouvant être reliée à un module de détection de suivi oculaire (ou « eye tracking » en anglais) afin de détecter la fréquence (et optionnellement l'amplitude) des mouvements oculaires de l'utilisateur. Une telle caméra intérieure peut équiper un système dit « DMS » (pour « Driver Monitoring System ») éventuellement préexistant dans le véhicule et servant notamment à détecter un endormissement du conducteur par exemple.

La présente invention vise aussi un programme informatique comportant des instructions pour la mise en oeuvre du procédé ci-avant, lorsque ces instructions sont exécutées par un processeur. Elle vise aussi de façon équivalente un support mémoire non transitoire apte à stocker durablement (ou non durablement) un tel programme informatique.

### Brève description des dessins

D'autres caractéristiques, détails et avantages apparaîtront à la lecture de la description détaillée ci-après, et à l'analyse des dessins annexés, sur lesquels :
**Fig. 1**
   [Fig. 1] montre un schéma d'un système de détection d'un état de conduite active ou non d'un utilisateur dans un habitacle de véhicule automobile selon un mode de réalisation de l'invention.
**Fig. 2**
   [Fig. 2] montre très schématiquement un mouvement oculaire d'un utilisateur, formant des allers-retours n1, n2, n3, n4, etc., a priori de façon aléatoire, mais dont la fréquence (et accessoirement la tendance à être proche d'une ligne horizontale d'une part et à avoir une amplitude moyenne supérieure à un seuil AMP d'autre part) caractérisent un utilisateur regardant défiler un paysage pendant un trajet automobile, sans être ainsi en état de conduite active du véhicule,
**Fig. 3**

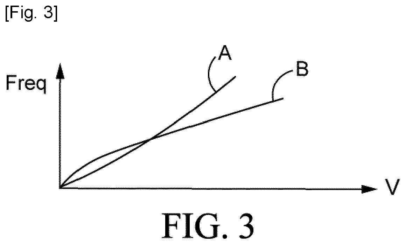

   [Fig. 3] compare schématiquement les fréquences de mouvements oculaires (ici en fonction de la vitesse du véhicule) de deux utilisateurs différents A et B,
**Fig. 4**

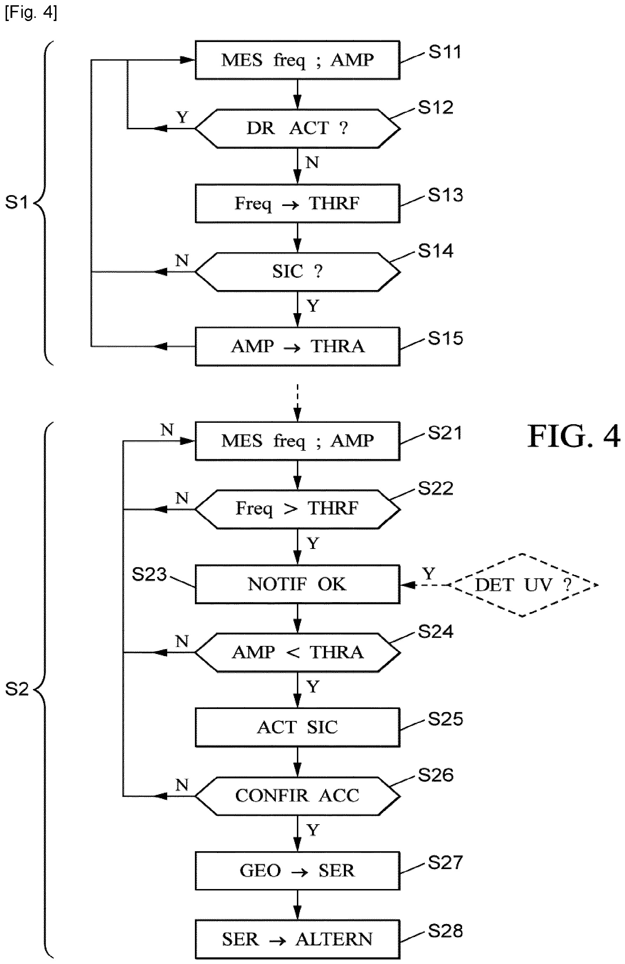

   [Fig. 4] illustre les étapes à titre d'exemple d'un procédé au sens de la présente divulgation.

### Description des modes de réalisation

Les dessins et la description ci-après contiennent, pour l'essentiel, des éléments de caractère certain. Ils pourront donc non seulement servir à mieux faire comprendre la présente divulgation, mais aussi contribuer à sa définition, le cas échéant.

On se réfère à la figure 1 pour décrire tout d'abord un dispositif de détection d'un état d'un passager d'un véhicule automobile comme conduisant activement ou non le véhicule.

Le dispositif comporte une pluralité de capteurs 31, 32, 33, 34, implantés, dans l'exemple décrit, sur la monture d'une paire de lunettes connectée 1. Ces capteurs sont en effet connectés à un module de communication 13 implanté dans la monture et capable d'envoyer des signaux de mesures issus de ces capteurs à un circuit de traitement 14 que comporte en outre le dispositif. En particulier, le capteur 31 est une première caméra apte à suivre le mouvement oculaire (de l'oeil gauche ici de l'utilisateur), et le capteur 32 est une deuxième caméra apte à suivre le mouvement oculaire de l'oeil gauche de l'utilisateur. En particulier, ces caméras 31, 32 et le circuit de traitement 14 sont ensemble capables de mesurer un déplacement de la rétine de chaque oeil de l'utilisateur relativement au cadre formé par le pourtour de l'oeil. A des fins de représentation, la figure 1 montre le pourtour 10 de l'oeil gauche, le pourtour 11 de l'oeil droit, ainsi que la position de la pupille 12 de l'oeil gauche au sein d'un quadrillage numéroté de A à J pour les colonnes et de 1 à 5 pour les lignes. La position de la pupille de l'oeil droit est également représentée. Le quadrillage est un découpage virtuel de la surface dans laquelle les pupilles évoluent lorsqu'elles sont en mouvement.

Les capteurs 31, 32 peuvent opérer par exemple dans l'infrarouge et peuvent comporter chacun une diode d'émission de lumière infrarouge et un photodétecteur infrarouge. La diode d'émission de lumière infrarouge est sélectionnée pour présenter des caractéristiques sans danger de détérioration de la vision humaine. La diode d'émission de lumière infrarouge est configurée pour émettre un faisceau infrarouge dans la direction de la pupille d'un des deux yeux, et le photodétecteur infrarouge est apte à détecter une réflexion du faisceau infrarouge renvoyée par la cornée de l'oeil.

Bien entendu, dans une variante simplifiée, une seule caméra visant un seul oeil de l'utilisateur peut être prévue. En outre, alternativement à un montage sur une paire de lunettes connectées, la caméra peut être montée dans l'habitacle par exemple dans le cadre d'une surveillance DMS comme indiqué précédemment.

Le circuit de traitement 14 comporte une interface de communication 16 notamment avec le module 13 (via par exemple un réseau local 15), ainsi qu'un processeur 17 et une mémoire de travail 18 coopérant avec le processeur 17. Le circuit de traitement 14 est alors configuré pour déterminer la position de la pupille dans le quadrillage virtuel à partir de l'intensité et du spectre des faisceaux infrarouge réfléchis et captés par chacun les photo-détecteurs. Le circuit de traitement 14 est en outre configuré pour horodater les positions des pupilles au fur et à mesure qu'elles sont captées. Pour ce faire, le circuit de traitement (et plus particulièrement son processeur 17) peut comporter en outre une horloge ou des moyens d'accès à des données d'horloge extérieure (non représentés).

Alternativement à une communication entre le module 13 et le circuit de traitement 14 via par exemple un réseau local 15 dans l'habitacle, le circuit de traitement 14 peut être directement implanté dans la monture de lunettes 1 et être connecté directement aux capteurs 31 à 34. En revanche, dans l'exemple illustré, l'interface de communication 16 du circuit de traitement 14 est préférablement connectée, directement ou indirectement (via le réseau local 15 ou via un réseau étendu WAN), à un appareil communiquant SM (de type smartphone ou autre) à disposition de l'utilisateur, notamment pour filtrer ou non des notifications destinées à l'utilisateur. En outre, dans l'exemple représenté, l'interface de communication 16 du circuit de traitement 14 est connectée, préférablement via le réseau étendu WAN, à un serveur distant SER-DB qui tient à jour des informations sur des trajets à risque et en particulier susceptibles de donner aux passagers un mal des transports. À cet effet, ce serveur peut comporter au moins une interface de communication et une mémoire de stockage (non représentées).

En outre, la monture de lunettes connectée 1 peut comporter également un capteur 33 de type accéléromètre pour confirmer qu'un trajet en cours est à risque comme détaillé plus loin, ce capteur étant connecté aussi au circuit de traitement 14 (via éventuellement le module de communication 13). En outre, la monture de lunettes connectée 1 peut comporter également un capteur 34 de détection de rayonnement UV pour comparer un rayonnement courant à un seuil et déclencher une action si le rayonnement courant est supérieur au seuil. Ce capteur est connecté aussi au circuit de traitement 14 (via éventuellement le module de communication 13).

La figure 2 illustre les mouvements oculaires au cours du temps et en particulier des allers-retours n1, n2, n3, n4 etc. de la pupille de l'oeil, ces allers-retours étant caractéristiques d'un passager regardant un paysage défilant, et n'étant pas concentré fixement sur la route comme le serait un conducteur actif du véhicule. Par ailleurs, les mouvements sont en moyenne horizontaux (ou s'écarte peu d'une ligne sensiblement horizontale) et sont d'une amplitude moyenne AMP plus grande qu'une amplitude habituelle de mouvements oculaires d'un conducteur actif de véhicule. Ainsi, le circuit de traitement 14 est programmé au moins pour :
- compter le nombre d'allers-retours n1, n2, etc. par une unité de temps (par exemple en une minute) qui ont une amplitude AMP significative, et
- en déduire une fréquence freq de ces mouvements oculaires caractéristiques.

Cette fréquence freq peut varier notamment en fonction de la vitesse courante v du véhicule automobile. Toutefois, ces données telles que la fréquence freq et l'amplitude AMP sont surtout propres à chaque individu. On a représenté à titre purement illustratif sur la figure 3 une variation (ici en fonction de la vitesse v du véhicule) de la fréquence freq de mouvements oculaires pour deux individus respectifs A et B.

Ainsi, en référence maintenant à la figure 4, il est proposé une première étape générale S1 d'apprentissage par intelligence artificielle (par exemple un réseau de neurones, profond par exemple) de paramètres caractéristiques de l'utilisateur tels que la fréquence freq (notamment en fonction de la vitesse v) et de l'amplitude moyenne AMP. En effet, à titre d'exemples non limitatifs, l'intelligence artificielle peut être mise en oeuvre par un apprentissage « en profondeur » (appelé en anglais « deep learning »), par un réseau de neurones entrainé, ou par de l'apprentissage « machine » (de l'anglais « machine learning »). On peut définir un « réseau de neurones » généralement par un ensemble de fonctions de transfert dont les coefficients sont modifiés progressivement (au fur et à mesure de l'apprentissage) en fonction d'une nouvelle acquisition de séquence de positions horodatées des pupilles et marquée comme étant caractéristique d'un scénario à reconnaitre.

Cette étape générale d'apprentissage S1 précède alors une utilisation courante S2 de l'intelligence artificielle pour détecter un état de conduite active (effective) de l'utilisateur et éventuellement s'il subit un mal des transports.

Ainsi, une première étape S11 consiste à mettre en oeuvre les capteurs 31 et 32 pour mesurer répétitivement l'amplitude AMP de mouvements oculaires d'un utilisateur donné et leur fréquence freq (en fonction de la vitesse v du véhicule par exemple). Ces mesures sont marquées (ou « taguées ») par un identifiant dans une base de données en fonction d'une situation réelle, effective, de l'utilisateur. Il peut être renseigné typiquement que l'utilisateur est (sortie Y de l'étape S12) ou n'est pas dans un état de conduite active. Typiquement, s'il n'est pas dans un état de conduite active (sortie N de l'étape S12), il peut être déterminé sa fréquence de mouvements oculaires en cours à l'étape S13 et les étapes S11 à S13 sont menées répétitivement jusqu'à déterminer une fréquence moyenne minimale THRF au-delà de laquelle l'utilisateur est de façon certaine dans un état où il ne conduit pas activement le véhicule (simple passager ou véhicule en mode de conduite autonome au-delà de 80 %). Il est demandé en outre à l'utilisateur à l'étape S14 s'il ne ressent pas un mal des transports (répétitivement) et si tel est le cas (sortie Y du test S14), la mesure courante d'amplitude AMP des mouvements oculaires peut être taguée dans la base d'apprentissage à l'étape S15 comme étant un seuil minimal THRA en-dessous duquel l'utilisateur commence à ressentir un mal des transports. Il a été observé en effet qu'une amplitude de mouvements oculaires inférieurs à un seuil mais caractérisant néanmoins une personne regardant le paysage défiler, était en général corrélée à un mal des transports que subit cette personne.

Bien entendu, ces étapes sont répétées plusieurs fois pour tenir à jour à des seuils de fréquence THRF et d'amplitude THRA propres à l'utilisateur (respectivement au-delà duquel (THRF) il n'est pas en état de conduite active, et en-dessous duquel (THRA) il commence à ressentir un mal des transports).

On se réfère maintenant à la deuxième étape générale S2 de la figure 4 pour décrire une mise en oeuvre courante du procédé, une fois que les seuils de fréquence THRF et d'amplitude THRA ont été déterminés pour un utilisateur donné.

A l'étape S21, on mesure, sur cet utilisateur, la fréquence courante de ses mouvements oculaires et leur amplitude.
A l'étape S22, si cette fréquence est supérieure au seuil THRF (flèche Y en sortie du test S22), alors il peut être déterminé avec certitude que l'utilisateur n'est pas dans un état de conduite active et il est possible ainsi, à l'étape S23, de lui transmettre des notifications via par exemple son appareil communiquant (de type smartphone ou autre). Ainsi, si le smartphone SM comporte un module informatique :
- pour détecter un état de mobilité dont la vitesse est supérieure à un seuil (ce qui signifie que l'utilisateur est dans un véhicule en mouvement et qu'il est susceptible de conduire ce véhicule), et
- bloquer alors toute notification entrante (à titre de sécurité),
à l'étape S23, il est possible de désactiver ce module du smartphone et autoriser ainsi ce dernier à recevoir et afficher des notifications entrantes.

Par exemple, une notification entrante possible peut consister en une alerte pour un rayonnement UV à l'intérieur de l'habitacle, détecté comme étant excessif par le capteur 34 à l'étape S29 (flèche Y en sortie du test S29). Dans ce cas et comme précédemment décrit, il peut être prévu en outre une action spécifique dans l'habitacle telle que par exemple une aspersion par brumisation d'eau en direction de l'utilisateur, ou autres.

Par ailleurs et optionnellement, l'amplitude AMP des mouvements oculaires de l'utilisateur peut être suivie pour déterminer si elle devient inférieure à un seuil HRTA déterminé à l'étape générale S1, ce qui signifie que l'utilisateur est détecté à l'étape S24 comme souffrant d'un mal des transports. Dans ce cas (flèche Y en sortie du test S24), il est prévu de déclencher une action dans l'habitacle à l'étape S25 pour soulager l'utilisateur. Il peut s'agir typiquement :
- d'un ralentissement de l'allure du véhicule, notamment dans les virages (en cas de conduite autonome),
- d'une modification des conditions thermiques dans l'habitacle pour l'utilisateur,
- d'une diffusion de senteurs spécifiques pouvant le soulager,
- d'une projection d'un hologramme pour attirer l'attention de l'utilisateur, ou autre.

Éventuellement, le capteur 33, de type accéléromètre, peut confirmer la présence de chaos que subit l'utilisateur dans le véhicule, à l'étape S26. Dans ce cas, une donnée de géolocalisation courante du véhicule peut être transmise, à l'étape S27, au serveur SER-DB présenté ci-avant en référence à la figure 1, pour stocker cette géolocalisation courante (ou plus globalement le trajet en cours qu'empreinte le véhicule) avec un identifiant de risque de mal des transports. Cette donnée de géolocalisation courante peut être déterminée par une puce GPS que peut comporter le circuit de traitement 14, ou peut être obtenue alternativement par communication avec le smartphone SM de l'utilisateur (comportant habituellement un moyen d'obtention d'une donnée de géolocalisation par une puce GPS ou par triangulation dans le réseau cellulaire). Cet identifiant peut permettre de noter le parcours avec un score de risque de mal des transports qui peut être fonction de la fréquence des chaos détectés et/ou de leur amplitude.

Éventuellement, le circuit de traitement 14 (par exemple via le smartphone SM) peut solliciter alors le serveur SER-DB à l'étape S28 pour obtenir un parcours alternatif qui peut alors s'afficher sur un écran d'un dispositif de navigation ou autre, connecté au circuit de traitement 14.

Bien entendu, la présente invention ne se limite pas aux formes de réalisation décrites ci-avant à titre d'exemples ; elle s'étend à d'autres variantes selon les revendications annexées.

Par exemple, on a décrit ci-avant un procédé dans lequel l'intelligence artificielle permet de définir une signature spécifique des mouvements oculaires d'un individu qui n'est pas en état de conduite active. Bien entendu, il est possible de définir d'abord une signature moyenne d'un panel d'utilisateurs (dans une première étape S1 générale S1) et de l'appliquer ensuite, dans une étape courante S2, à un utilisateur donné. Par ailleurs, il est possible d'affiner la signature des mouvements oculaires d'un individu donné en confirmant, pendant l'étape courante S2, que l'utilisateur est réellement ou non en état de conduite active. A titre d'exemple, une notification peut être transmise sur un écran quelconque de l'habitacle du véhicule ou sur le smartphone de l'utilisateur et s'il n'y répond pas, cela signifie qu'il est effectivement un état de conduite active.

Par ailleurs, les capteurs 31 à 34 peuvent être regroupés sur une monture de lunettes connectées comme décrit ci-avant, ou alternativement être dispersés dans l'habitacle du véhicule en étant néanmoins connectés au circuit de traitement 14.

## Revendications

1. **Procédé** de détermination d'au moins un état d'un utilisateur dans un habitacle de véhicule automobile, en tant que personne conduisant activement ou non le véhicule, dans lequel on prévoit un dispositif capteur (31 , 32) de mouvements oculaires de l'utilisateur le procédé comportant :
- alimenter une intelligence artificielle avec des données issues du dispositif capteur pour reconnaître au moins une fréquence de mouvements oculaires de l'utilisateur, une fréquence de mouvements oculaires au-delà d'un premier seuil caractérisant une visualisation par l'utilisateur d'un paysage défilant, et se distinguant d'une concentration de regard d'un conducteur de véhicule,
- déterminer une fréquence courante de mouvements oculaires de l'utilisateur et comparer la fréquence courante au premier seuil, et
- si la fréquence courante est supérieure au premier seuil, déclencher un signal de notification à destination de l'utilisateur.

2. Procédé selon la revendication 1, dans lequel, si la fréquence (THRF) courante est supérieure au premier seuil, l'utilisateur est déterminé comme simple passager ou conducteur du véhicule sous un mode de conduite autonome à un niveau supérieur ou égal à 80%.

3. Procédé selon l'une des revendications précédentes, dans lequel le signal de notification est transmis à un appareil (SM) communiquant de l'utilisateur.

4. Procédé selon la revendication 3, dans lequel l'appareil communiquant est configuré pour :
- déterminer si l'utilisateur est dans un véhicule en mouvement, et
- dans ce cas, filtrer des notifications entrantes sur l'appareil communiquant, le procédé comportant en outre :
- déterminer si l'utilisateur n'est pas en situation de conduite active, et dans ce cas désactiver le filtrage des notifications entrantes sur l'appareil communiquant.

5. Procédé selon l'une des revendications 3 et 4, comportant en outre :
- déterminer une position courante du véhicule, et
- déclencher des signaux de notifications pertinentes en fonction de la position courante du véhicule, à destination de l'appareil de l'utilisateur.

6. Procédé selon l'une des revendications précédentes, dans lequel le dispositif capteur (31, 32) mesure en outre une amplitude (AMP) de mouvements oculaires, et le procédé comporte en outre :
- comparer une amplitude courante à une amplitude seuil (THRA) et
- si l'amplitude courante (AMP) est inférieure à l'amplitude seuil (THRA), déterminer une situation de mal des transports que subit l'utilisateur, et déclencher une action dans le véhicule pour soulager l'utilisateur.

7. Procédé selon la revendication 6, comportant :
- alimenter une intelligence artificielle avec des données issues du dispositif capteur pour reconnaître au moins une amplitude de mouvements oculaires de l'utilisateur, l'utilisateur étant en situation de mal des transports lorsque l'amplitude de mouvements oculaires mesurée est inférieure à ladite amplitude seuil.

8. Procédé selon l'une des revendications 6 et 7, dans lequel l'action dans le véhicule comporte au moins l'un des éléments parmi :
- une réduction de vitesse du véhicule au moins dans des virages que prend le véhicule,
- une aspersion de senteurs dans l'habitacle du véhicule,
- une modification des conditions thermiques dans l'habitacle,
- une projection d'un scénario lumineux dans l'habitacle.

9. Procédé selon l'une des revendications 6 à 8, comportant en outre :
- obtenir des coordonnées de géolocalisation courantes du véhicule pour identifier un parcours en cours du véhicule comme étant facteur de risque de déclenchement de mal des transports, et
- transmettre les coordonnées de géolocalisation courantes à un serveur de base de données avec un identifiant de mal des transports.

10. Procédé selon la revendication 9, dans lequel le serveur distant est capable de :
- recevoir des coordonnées de géolocalisations depuis une flotte de véhicules, avec un identifiant de mal des transports,
- estimer pour une pluralité de parcours possibles d'un point à un autre, des scores respectifs de risque de déclenchement de mal des transports, et
- transmettre à un ou plusieurs véhicules des données pour déterminer un parcours alternatif à un parcours à score élevé de risque de déclenchement de mal des transports.

11. Procédé selon l'une des revendications 9 et 10, dans lequel on prévoit en outre un accéléromètre (33) pour estimer une fréquence courante de cahots du véhicule, un parcours étant confirmé comme à risque de déclenchement de mal des transports si la fréquence courante de cahots dépasse une fréquence seuil.

12. Procédé selon l'une des revendications précédentes, dans lequel on prévoit en outre un capteur (34) de rayonnement UV et, en cas de détection d'une signature de rayonnement UV supérieure à un rayonnement seuil, ladite notification à destination de l'utilisateur comporte un message de sensibilisation au rayonnement UV.

13. **Dispositif** comportant un circuit de traitement et au moins un capteur de mouvements oculaires pour la mise en oeuvre du procédé selon l'une des revendications précédentes.

14. Dispositif selon la revendication 13, dans lequel le dispositif capteur de mouvements oculaires de l'utilisateur est monté sur une paire de lunettes connectée que porte l'utilisateur.

15. Dispositif selon la revendication 13, dans lequel le dispositif capteur de mouvements oculaires de l'utilisateur est monté sur une caméra aménagée dans l'habitacle du véhicule.

16. **Programme informatique** comportant des instructions pour la mise en oeuvre du procédé selon l'une des revendications 1 à 12, lorsque lesdites instructions sont exécutées par un processeur.

## Patentansprüche

1. **Verfahren** zur Bestimmung mindestens eines Zustands eines Benutzers in einem Fahrgastraum eines Motorfahrzeugs, als eine Person, die das Fahrzeug aktiv fährt oder nicht,
wobei eine Sensorvorrichtung (31, 32) für Augenbewegungen des Benutzers vorgesehen ist,
wobei das Verfahren Folgendes umfasst:
- Versorgen einer künstlichen Intelligenz mit Daten aus der Sensorvorrichtung, um mindestens eine Augenbewegungsfrequenz des Benutzers zu erkennen, wobei eine Augenbewegungsfrequenz oberhalb eines ersten Schwellenwerts angibt, dass der Benutzer eine vorbeiziehende Landschaft betrachtet, und sich von einem konzentrierten Blick eines Fahrzeugfahrers unterscheidet,
- Bestimmen einer aktuellen Augenbewegungsfrequenz des Benutzers und Vergleichen der aktuellen Frequenz mit dem ersten Schwellenwert, und
- wenn die aktuelle Frequenz größer als der erste Schwellenwert ist, Auslösen eines Benachrichtigungssignals für den Benutzer.

2. Verfahren nach Anspruch 1, wobei, wenn die aktuelle Frequenz (THRF) größer als der erste Schwellenwert ist, bestimmt wird, dass der Benutzer ein einfacher Mitfahrer oder ein Fahrer des Fahrzeugs in einem autonomen Fahrmodus mit einem Grad von größer als oder gleich 80 % ist.

3. Verfahren nach einem der vorhergehenden Ansprüche, wobei das Benachrichtigungssignal an ein Kommunikationsgerät (SM) des Benutzers übertragen wird.

4. Verfahren nach Anspruch 3, wobei das Kommunikationsgerät zu Folgendem konfiguriert ist:
- Bestimmen, ob sich der Benutzer in einem sich bewegenden Fahrzeug befindet, und
- in diesem Fall, Filtern von auf dem Kommunikationsgerät eingehenden Benachrichtigungen,
wobei das Verfahren ferner Folgendes umfasst:
- Bestimmen, ob sich der Benutzer nicht in einer aktiven Fahrsituation befindet, und in diesem Fall, Deaktivieren des Filterns der auf dem Kommunikationsgerät eingehenden Benachrichtigungen.

5. Verfahren nach einem der Ansprüche 3 und 4, das ferner Folgendes umfasst:
- Bestimmen einer aktuellen Position des Fahrzeugs und
- Auslösen entsprechender Benachrichtigungssignale in Abhängigkeit von der aktuellen Position des Fahrzeugs für das Gerät des Benutzers.

6. Verfahren nach einem der vorhergehenden Ansprüche, wobei die Sensorvorrichtung (31, 32) ferner eine Augenbewegungsamplitude (AMP) misst und das Verfahren ferner Folgendes umfasst:
- Vergleichen einer aktuellen Amplitude mit einer Schwellenamplitude (THRA), und
- wenn die aktuelle Amplitude (AMP) kleiner als die Schwellenamplitude (THRA) ist, Bestimmen einer durch Reisekrankheit gekennzeichneten Situation des Benutzers und Auslösen einer Aktion in dem Fahrzeug, um dem Benutzer zu helfen.

7. Verfahren nach Anspruch 6, das Folgendes umfasst:
- Versorgen einer künstlichen Intelligenz mit Daten aus der Sensorvorrichtung, um mindestens eine Augenbewegungsamplitude des Benutzers zu erkennen, wobei sich der Benutzer in einer durch Reisekrankheit gekennzeichneten Situation befindet, wenn die gemessene Augenbewegungsamplitude kleiner als die Schwellenamplitude ist.

8. Verfahren nach einem der Ansprüche 6 und 7, wobei die Aktion in dem Fahrzeug mindestens eines der folgenden Elemente umfasst:
- eine Reduzierung der Geschwindigkeit des Fahrzeugs mindestens in Kurven, die das Fahrzeug nimmt,
- ein Versprühen von Düften in dem Fahrgastraum des Fahrzeugs,
- eine Veränderung der Temperaturbedingungen in dem Fahrgastraum,
- ein Projizieren eines Lichtszenarios in dem Fahrgastraum.

9. Verfahren nach einem der Ansprüche 6 bis 8, das ferner Folgendes umfasst:
- Erhalten von aktuellen Geolokalisierungskoordinaten des Fahrzeugs, um zu identifizieren, dass eine aktuelle Route des Fahrzeugs ein Risikofaktor für das Auslösen einer Reisekrankheit ist, und
- Übertragen der aktuellen Geolokalisierungskoordinaten an einen Datenbankserver zusammen mit einer Reisekrankheitskennung.

10. Verfahren nach Anspruch 9, wobei der entfernte Server zu Folgendem fähig ist:
- Empfangen von Geolokalisierungskoordinaten von einer Fahrzeugflotte zusammen mit einer Reisekrankheitskennung,
- Ermitteln, für eine Vielzahl von möglichen Routen von einem Punkt zu einem anderen, jeweiliger Scores für das Risiko der Auslösung einer Reisekrankheit und
- Übertragen von Daten an ein oder mehrere Fahrzeuge, um eine Route zu bestimmen, die zu einer Route mit hohem Score für das Risiko der Auslösung einer Reisekrankheit alternativ ist.

11. Verfahren nach einem der Ansprüche 9 und 10, wobei ferner ein Beschleunigungsmesser (33) vorgesehen ist, um eine aktuelle Stoßfrequenz des Fahrzeugs zu ermitteln, wobei bestätigt wird, dass eine Route ein Risiko der Auslösung einer Reisekrankheit besitzt, wenn die aktuelle Stoßfrequenz eine Schwellenfrequenz überschreitet.

12. Verfahren nach einem der vorhergehenden Ansprüche, wobei ferner ein UV-Strahlungssensor (34) vorgesehen ist und im Fall der Detektion einer UV-Strahlungssignatur, die größer als eine Schwellenstrahlung ist, die Benachrichtigung für den Benutzer eine in Bezug auf UV-Strahlung sensibilisierende Nachricht umfasst.

13. **Vorrichtung,** die eine Verarbeitungsschaltung und mindestens einen Augenbewegungssensor für die Umsetzung des Verfahrens nach einem der vorhergehenden Ansprüche umfasst.

14. Vorrichtung nach Anspruch 13, wobei die Sensorvorrichtung für Augenbewegungen des Benutzers an einer Datenbrille, die der Benutzer trägt, angebracht ist.

15. Vorrichtung nach Anspruch 13, wobei die Sensorvorrichtung für Augenbewegungen des Benutzers an einer Kamera, die im Fahrgastraum des Fahrzeugs eingerichtet ist, angebracht ist.

16. **Computerprogramm,** das Anweisungen zur Umsetzung des Verfahrens nach einem der Ansprüche 1 bis 12 umfasst, wenn die Anweisungen von einem Prozessor ausgeführt werden.

## Claims

1. **Method** for determining at least one state of a user in a motor-vehicle passenger compartment, regarding whether they are actively driving the vehicle or not, wherein an eye-movement sensor device (31, 32) for sensing eye movements of the user is provided, the method comprising:
- feeding an artificial intelligence with data generated by the sensor device with a view to recognizing at least one eye-movement frequency of the user, an eye-movement frequency above a first threshold being characteristic of the user viewing a landscape passing by, and being distinguishable from the concentrated gaze of a vehicle driver,
- determining a current eye-movement frequency of the user and comparing the current frequency with the first threshold, and
- if the current frequency is higher than the first threshold, triggering a notification signal intended for the user.

2. Method according to Claim 1, wherein, if the current frequency (THRF) is higher than the first threshold, it is determined that the user is simply a passenger, or a driver of the vehicle in an autonomous driving mode at a level higher than or equal to 80%.

3. Method according to one of the preceding claims, wherein the notification signal is transmitted to a communication apparatus (SM) of the user.

4. Method according to Claim 3, wherein the communication apparatus is configured to:
- determine whether the user is in a moving vehicle, and
- in this case, filter incoming notifications to be delivered to the communication apparatus, the method further comprising:
- determining whether the user is not in an active driving situation, and in this case deactivating filtering of incoming notifications to be delivered to the communication apparatus.

5. Method according to one of Claims 3 and 4, further comprising:
- determining a current position of the vehicle, and
- triggering, depending on the current position of the vehicle, relevant notification signals intended for the device of the user.

6. Method according to one of the preceding claims, wherein the sensor device (31, 32) further measures an eye-movement amplitude (AMP), and the method further comprises:
- comparing a current amplitude with a threshold amplitude (THRA), and
- if the current amplitude (AMP) is lower than the threshold amplitude (THRA), determining that the user is suffering from an episode of motion sickness, and triggering an action in the vehicle to provide relief to the user.

7. Method according to Claim 6, comprising:
- feeding an artificial intelligence with data generated by the sensor device with a view to recognizing at least one eye-movement amplitude of the user, the user having an episode of motion sickness when the measured eye-movement amplitude is lower than said threshold amplitude.

8. Method according to one of Claims 6 and 7, wherein the action in the vehicle comprises at least one of the elements among:
- decreasing the speed of the vehicle at least in bends that the vehicle takes,
- spraying scents into the passenger compartment of the vehicle,
- modifying the thermal conditions in the passenger compartment,
- projecting a light scenario into the passenger compartment.

9. Method according to one of Claims 6 to 8, further comprising:
- obtaining current geolocation coordinates of the vehicle with a view to identifying a current route of the vehicle as being a risk factor for onset of motion sickness, and
- transmitting the current geolocation coordinates to a database server with a motion-sickness identifier.

10. Method according to Claim 9, wherein the remote server is capable of:
- receiving geolocation coordinates from a fleet of vehicles, with a motion-sickness identifier,
- estimating, for a plurality of possible routes from one point to another, respective motion-sickness-onset-risk scores, and
- transmitting, to one or more vehicles, data with a view to determining an alternative route to a route with a high motion-sickness-onset-risk score.

11. Method according to one of Claims 9 and 10, wherein an accelerometer (33) is further provided with a view to estimating a current vehicle-jolt frequency, a route being confirmed a motion-sickness-onset risk if the current jolt frequency exceeds a threshold frequency.

12. Method according to one of the preceding claims, wherein a UV-radiation sensor (34) is further provided and, in case of detection of a UV-radiation signature higher than a radiation threshold, said notification intended for the user comprises a message raising consciousness of UV radiation.

13. **Device** comprising a processing circuit and at least one eye-movement sensor for implementing the method according to one of the preceding claims.

14. Device according to Claim 13, wherein the eye-movement sensor device for sensing eye movements of the user is mounted on a connected pair of glasses worn by the user.

15. Device according to Claim 13, wherein the eye-movement sensor device for sensing eye movements of the user is mounted on a camera arranged in the passenger compartment of the vehicle.

16. **Computer program** comprising instructions for implementing the method according to one of Claims 1 to 12, when said instructions are executed by a processor.
